(19) Europäisches Patentamt
European Patent Office
Office européen des brevets

(11) Numéro de publication: **0 097 072**
**A1**

(12) # DEMANDE DE BREVET EUROPEEN

(21) Numéro de dépôt: **83401047.2**

(22) Date de dépôt: **26.05.83**

(51) Int. Cl.³: **C 07 C 147/14**, C 07 D 295/12, A 61 K 31/16, A 61 K 31/395

(30) Priorité: **04.06.82 FR 8209803**

(43) Date de publication de la demande: **28.12.83**
**Bulletin 83/52**

(84) Etats contractants désignés: **AT BE CH DE FR GB IT LI LU NL SE**

(71) Demandeur: **LABORATOIRE L. LAFON Société anonyme dite:, 1 rue Georges Médéric, F-94701 Maisons-Alfort (FR)**

(72) Inventeur: **Lafon, Louis, 5 rue de l'Alboni, F-75016 Paris (FR)**

(74) Mandataire: **Clisci, Serge et al, CABINET BEAU DE LOMENIE 55 rue d'Amsterdam, F-75008 Paris (FR)**

(54) **Nouveaux dérivés de benzylsulfinylacétamide, utilisation en thérapeutique et procédé de préparation.**

(57) La présente invention concerne en tant que produits industriels nouveaux, les méthoxybenzylsulfinyl-acétamides de formule

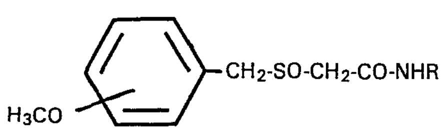

I

(où R est H ou $CH_2CH_2B$, B représentant un groupe diéthylamino, diméthylamino, morpholino, pipéridino, pyrrolidino, hexaméthylène-imino ou pipérazino) et leurs sels quand R comporte un reste basique.

EP 0 097 072 A1

Nouveaux dérivés de benzylsulfinylacétamide, utilisation en thérapeutique et procédé de préparation.

La présente invention concerne en tant que produits industriels de nouveaux dérivés de benzylsulfinylacétamide. Elle concerne également l'utilisation de ces nouveaux produits en tant que médicaments, ainsi que leur procédé de préparation.

Les produits selon l'invention qui appartiennent à la famille des dérivés de benzylsulfinylacétamide, sont caractérisés en ce qu'ils sont choisis parmi l'ensemble constitué par

(i) les méthoxybenzylsulfinyl-acétamides répondant à la formule générale

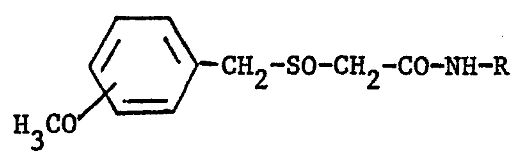

(I)

[où R représente un atome d'hydrogène ou un groupe $CH_2CH_2B$ (où B est diméthylamino-diéthylamino, morpholino, pipéridino, pyrrolidino, hexaméthylèneimino ou pipérazino)] ; et,

(ii) leurs sels d'addition d'acide quand le groupe R comporte un reste basique.

Par sels d'addition d'acide on entend ici les sels d'addition obtenus par réaction de I (quand R est $CH_2CH_2B$) avec des acides minéraux et organiques. Parmi les sels d'addition d'acide non toxiques qui conviennent selon l'invention on peut notamment mentionner ceux qui sont obtenus à partir des acides chlorhydrique, bromhydrique, nitrique, sulfurique, acétique, formique, propionique, benzoïque, malique, citrique, ascorbique, tartrique, maléique, fumarique, aspartique, cinnamique, méthanesulfonique et paratoluènesulfonique.

Dans le Tableau I ci-après on a consigné de façon nullement limitative un certain nombre de composés selon l'invention. Les composés préférés sont ceux où le groupe méthoxy

est en position para du groupe benzyle et où R représente l'atome d'hydrogène ou un groupe ,ß-morpholinoéthyle ou ß-pipéridino-éthyle.

TABLEAU I

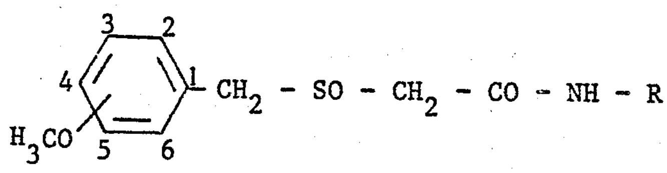

| Produit | No de code | position du groupe $OCH_3$ | R |
|---|---|---|---|
| Ex 1 | CRL 40569 | 4 | H |
| Ex 2 | CRL 40615 | 4 | 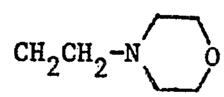 |
| Ex 3 | CRL 40615A | 4 | 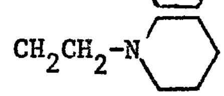 |
| Ex 4 | - | 3 | 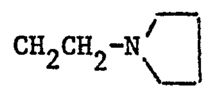 |
| Ex 5 | - | 3 | 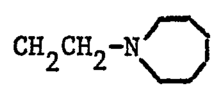 |
| Ex 5 | - | 4 | $CH_2CH_2-N(CH_3)_2$ |
| Ex 6 | - | 2 | $CH_2CH_2-N(C_2H_5)_2$ |
| Ex 7 | - | 4 | 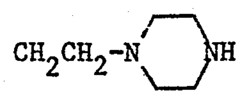 |

Les composés de formule I peuvent être préparés selon une méthode connue en soi par application de mécanismes réactionnels classiques. Le procédé que l'on préconise selon l'invention est schématisé comme suit :

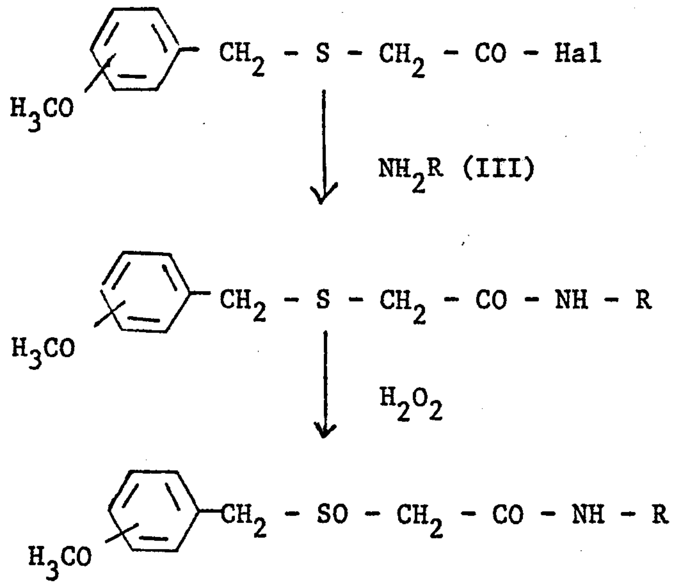


(II)

(IV)

(I)

et comprend

a) la réaction d'un halogénure d'acide de formule II (où Hal représente un atome d'halogène, de préférence Cl) avec une amine III (où R est défini comme ci-dessus), cette réaction étant effectuée en présence d'un excès d'amine III par rapport aux conditions stoechiométriques (de préférence à raison de 2 moles d'amine III pour 1 mole d'halogénure d'acide II) pour obtenir un dérivé benzylthioacétamide de formule IV, puis

b) l'oxydation dans de l'acide acétique dudit dérivé benzylthioacétamide IV au moyen de $H_2O_2$ à 110 - 130 volumes à une température inférieure à 50°C, et de préférence pendant au moins 1 heure à la température ambiante (15 - 25°C).

Les composés de formule I sont utiles en thérapeutique en tant que substances actives sur le SNC, et notamment en tant qu'agents sédatifs. Selon l'invention on préconise une composition thérapeutique qui comprend, en association avec un excipient physiologiquement acceptable au moins un composé de formule I ou l'un de ses sels d'addition non toxiques. Bien entendu les composés selon l'invention seront administrés à une dose pharmaceutiquement efficace.

D'autres avantages et caractéristiques de l'invention seront mieux compris à la lecture qui va suivre d'exemples de préparation nullement limitatifs, et du résumé des résultats des essais neuropsychopharmacologiques.

PREPARATION I

Obtention du 4-méthoxybenzylsulfinyl-acétamide

(Exemple 1 ; No de code : CRL 40569)

a) Chlorure de 4-méthoxybenzylthio-acétyle

Dans un tricol muni d'un réfrigérant et d'une ampoule de coulée, on place 42,4 g (0,2 mole) d'acide 4-méthoxybenzylthio-acétique dans 300 ml de benzène. On chauffe le mélange et l'on ajoute goutte à goutte 50 ml de chlorure de thionyle. Une fois l'addition terminée, on poursuit le reflux environ 1 h, refroidit, filtre, évapore le benzène et le chlorure de thionyle en excès. On recueille ainsi le chlorure de 4-méthoxybenzylthio-acétyle qui se présente sous la forme d'une huile orangée limpide.

b) 4-Méthoxybenzylthio-acétamide.

Dans un tricol d'1 litre muni d'un réfrigérant et d'une ampoule de coulée, on introduit 100 ml d'ammoniaque dans 110 ml d'eau, et l'on ajoute goutte à goutte le chlorure de 4-méthoxybenzylthio-acétyle précédemment obtenu et dissous dans environ 250 ml de chlorure de méthylène. Une fois l'addition terminée, on lave la phase organique avec une solution de soude diluée, sèche sur $Na_2SO_4$, évapore le solvant, reprend à l'éther diisopropylique. Le produit attendu cristallise ; on recueille 36,8 g (rendement = 87 %) de 4-méthoxybenzylthio-acétamide. $F_{inst}$ = 96°C.

c) CRL 40569

On met dans un ballon 31,65 g (0,15 mole)de 4-méthoxybenzylthio-acétamide obtenu comme indiqué ci-dessus, et ajoute 150 ml d'acide acétique et 20 ml de $H_2O_2$ à environ 110 volumes. On laisse le milieu réactionnel pendant environ 2 h à température ambiante (15 - 25°C) : des cristaux blancs apparaissent petit à petit. Ces cristaux sont filtrés et lavés à l'eau. Après recristallisation dans le mélange eau-éthanol (1 : 1) v/v on obtient 22 g (rendement =

70 %) de CRL 40569. $F_{inst}$ = 212°C.

PREPARATION II

Obtention du N-β-morpholinoéthyl-4-méthoxybenzylsulfinyl-acétamide.

(Exemple 2 ; No de code CRL 40615)

a) N-morpholinoéthyl-4-méthoxybenzylthio-acétamide.

Dans un tricol on place environ 17 g (0,127 mole) de N-β-aminoéthylmorpholine dans 100 ml d'eau distillée. On refroidit et ajoute goutte à goutte 14,7 g (0,0637 mole) de chlorure de 4-méthoxybenzylthio-acétyle. L'addition terminée on laisse le milieu réactionnel à 15 - 25°C pendant 1 h environ : il se forme un précipité qu'on essore et lave avec un peu d'eau. On recueille ainsi 14,4 g (rendement = 70 %) de N-morpholinoéthyl-4-méthoxybenzylthio-acétamide. $F_{inst}$ = 90°C.

b) CRL 40615

On place dans un ballon 12,96 g (0,04 mole) de N-morpholinoéthyl-4-méthoxybenzylthio-acétamide obtenu comme indiqué ci-dessus ; on ajoute 40 ml d'acide acétique et 4 ml de $H_2O_2$ à 110-130 volumes. On laisse le milieu réactionnel pendant 2 h à 15-25°C (en suivant le déroulement de la réaction d'oxydation par chromatographie). On évapore l'acide acétique, reprend le résidu huileux résultant avec de l'eau et ajoute de la soude diluée : le produit attendu précipite. On essore le précipité ainsi obtenu, le redissout dans $CH_2Cl_2$, filtre sur charbon, évapore $CH_2Cl_2$, puis par recristallisation dans l'acétate d'éthyle on obtient 9,1 g (rendement 67 %) de CRL 40615. $F_{inst}$ = 134°C.

Les résultats des essais qui ont été entrepris ont été résumés ci-après. Dans ces essais, le CRL 40569 (produit de l'exemple 1) en suspension dans une solution aqueuse de gomme arabique, et le CRL 40615 (produit de l'exemple 2) en solution dans de l'eau distillée ont été administrés par voie intrapéritonéale sous un volume de 20 ml/kg chez la souris mâle et sous un volume de 5 ml/kg chez le rat mâle.

TOXICITE

La DL-O (dose maximale non mortelle) chez la souris mâle par voie I.P. pour les CRL 40569 et CRL 40615 est supérieure à 1024 mg/kg.

COMPORTEMENT GLOBAL ET REACTIVITES

Des lots de 3 animaux par dose et par produit sont observés avant,puis 15 minutes, 30 minutes, 1 h, 2h, 3h et 24 h après administration des CRL 40569 et CRL 40615.

1°) Chez la souris

Avec le CRL 40569 à la dose de 512 mg/kg on note une diminution fugace de la réactivité au toucher, de la force et du tonus musculaire.

Avec le CRL 40615, aux doses de 512 mg/kg, 128 mg/kg et 32 mg/kg on observe une sédation pendant 15 à 30 minutes puis une excitation tardive qui se manifeste 24 h après administration.

2°) Chez le rat

A aucune des doses utilisées (256, 64, 16 et 4 mg/kg) le CRL 40569 n'a entraîné des modifications nettes du comportement et des réactivités.

A la dose de 256 mg/kg le CRL 40615 entraîne une diminution du tonus musculaire.

ACTION SUR LA MOTILITE SPONTANEE.

Une demi-heure après avoir reçu les produits à tester, les souris (6 par dose et par produit, 12 témoins pour chaque produit) sont placées en actimètre ou leur motilité est enregistrée pendant 30 minutes.

On observe que aux doses de 32, 128 et 512 mg/kg, le CRL 40569 provoque une diminution de la motilité spontanée.

On observe également que à forte dose (512 mg/kg), le CRL 40615 diminue la motilité spontanée.

INTERACTION AVEC L'AMPHETAMINE.

Des lots de 6 rats reçoivent une injection par voie I.P. de 2 mg/kg d'amphétamine une demi-heure après le CRL 40569.

On observe que à la dose de 256 mg/kg, le CRL 40569 diminue légèrement l'intensité des stéréotypies amphétaminiques.

ACTION SUR L'AGRESSIVITE INTERGROUPES.

Après avoir séjourné pendant 3 semaines de part et d'autre d'une cloison opaque séparant leur cage par le milieu, des groupes de 3 souris reçoivent le CRL 40615. Une demi-heure plus tard, les deux groupes d'une même cage sont réunis par retrait de la cloison et on note le nombre de combats qui surviennent en 10 minutes.

On constate que, notamment à la dose de 512 mg/kg I.P, le CRL 40615 diminue le nombre de combats.

REVENDICATIONS

1. Nouveaux dérivés de benzylsulfinylacétamide, caractérisés en ce qu'ils sont choisis parmi l'ensemble constitué par

(i) les méthoxybenzylsulfinyl-acétamides répondant à la formule générale

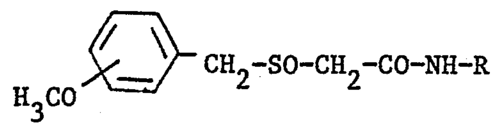 (I)

/ où R représente un atome d'hydrogène ou un groupe $CH_2CH_2B$ (où B est diméthylamino, diéthylamino, morpholino, pipéridino, pyrrolidino, hexaméthylèneimino ou pipérazino) /; et,

(ii) leurs sels d'addition d'acide quand le groupe R comporte un reste basique.

2. Nouveaux dérivés de benzylsulfinylacétamide selon la revendication 1, caractérisés en ce que le groupe $OCH_3$ est en position para sur le noyau phényle.

3. Nouveaux dérivés de benzylsulfinylacétamide selon la revendication 1, caractérisés en ce que R représente H, le groupe β-morpholinoéthyle ou β-pipéridinoéthyle.

4. 4-Méthoxybenzylsulfinyl-acétamide.

5. N- β-Morpholinoéthyl-4-méthoxybenzylsulfinyl-acétamide.

6. Composition thérapeutique caractérisée en ce qu'elle renferme, en association avec un excipient physiologiquement acceptable, au moins un dérivé de benzylsulfinyl-acétamide selon l'une quelconque des revendications 1 à 5.

7. Procédé de préparation d'un composé de formule I selon la revendication 1, dans lequel on condense un halogénure d'acide benzylthioacétique avec une amine puis oxyde le dérivé thio résultant, ledit procédé étant caractérisé en ce que

a) on fait réagir un halogénure de méthoxybenzylthio-acétyle de formule

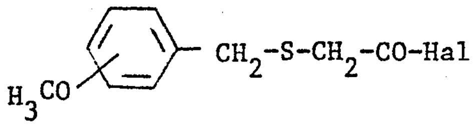

(II)

(où Hal représente un atome d'halogène, de préférence le chlore) avec une amine de formule

$$NH_2R \qquad \text{(III)}$$

(où R est défini comme ci-dessus) en excès (de préférence 2 moles de III pour 1 mole de II) pour obtenir un dérivé de méthoxybenzylthio-acétamide de formule

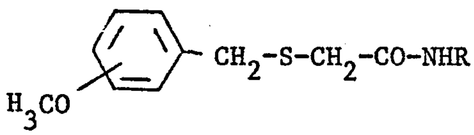

(IV)

puis,

b) on soumet le produit IV ainsi obtenu à une réaction d'oxydation dans de l'acide acétique au moyen de $H_2O_2$ à 110-130 volumes, à une température inférieure à 50°C pendant au moins 1 heure.

0097072

Office européen des brevets

## RAPPORT DE RECHERCHE EUROPEENNE

Numéro de la demande

EP 83 40 1047

### DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. 3) |
|---|---|---|---|
| Y | FR-A-2 385 693 (LAFON)<br>* Revendications *<br>--- | 1,6 | C 07 C 147/14<br>C 07 D 295/12<br>A 61 K 31/16<br>A 61 K 31/395 |
| Y | GB-A-1 121 027 (ICI)<br>* Revendications *<br>----- | 1,6 | |

DOMAINES TECHNIQUES RECHERCHES (Int. Cl. 3)

C 07 C 147/00
C 07 D 295/00
A 61 K 31/00

Le présent rapport de recherche a été établi pour toutes les revendications

| Lieu de la recherche | Date d'achèvement de la recherche | Examinateur |
|---|---|---|
| LA HAYE | 11-08-1983 | MOREAU J.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503. 03.82